# EUROPEAN PATENT APPLICATION

(11) **EP 2 875 811 A1**
(43) Date of publication of application: **27.05.2015**
(21) Application number: 13380056.5
(22) Date of filing: 26.11.2013
(51) Int. Cl.: A61K 31/185, A61P 27/02

(54) **Use of dobesilate for treating ocular haemorrhages**

(71) Applicant: Outeirino Miguez, Luis Antonio, 28014 Madrid (ES)
(72) Inventor: Outeirino Miguez, Luis Antonio, 28014 Madrid (ES)

(57) **Abstract**

Submacular and vitreous haemorrhage can be successfully treated with a single intravitreal injection of dobesilate without any adverse ocular effects related to treatment. These data support a new promising therapy for the treatment of haemorrhages of the eye.

## Description

### FIELD OF THE INVENTION

The invention describes a method of the use for dobesilate and pharmaceuticals acceptable salts thereof for treating submacular and vitreous haemorrhages. In the invention the pharmaceutically acceptable salt is dimethylamine

### BACKGROUND OF THE INVENTION

Submacular haemorrhage (SMH) is not an unusual case of acute central vision loss, particularly in older people. It may be caused by a number of conditions, most common of which is exudative age-related macular degeneration (AMD). In patients affected by this sort of macular degeneration, choroidal neovascularization extends into the subretinal space, producing in approximately 17% of the cases substantial bleeding, resulting in large haemorrhages in the subretinal space that detach the neurosensory retina from the supporting retinal pigment epithelial (RPE) layer, leading to substantial vision loss, because of a relatively fast process of extensive photoreceptor atrophy in the overlying neuroretina, and formation of macular scars (Poliner LS, et al. Ophthalmology. 1986; 93: 543-51. Avery RL et al. Retina. 1996; 16: 183-9. Zhao L, et al. Am J Pathol. 2011; 179: 1265-77. Hochman MA, et al. Surv Ophthalmol 1997, 42: 195-213). Patients with exudative AMD and SMH on initial presentation have more than a 50% chance of developing recurrent submacular bleedings within the first subsequent 3 years (Hwang JU et al. Retina. 2012; 32: 652-7).

Retinal diseases characterized by an excess of vessel proliferation are sometimes treated with antibodies that inhibit vascular endothelial growth factor (VEGF). Recently, anti-VEGF antibodies have been also proposed for the management of SMH (Stifter E et al. Am J Ophthalmol. 2007; 144: 886-92. McKibbin M et al. Eye (Lond). 2010; 24: 994-8). However, this therapy needs in some patients repeated injections and several months to clear SMH (Shienbaum G et al. Am J Ophthalmol. 2013; 155: 1009-13). In addition, SMH may occur following intravitreal anti-VEGF agents injection (Goverdhan SV & Lochhead J. Br J Ophthalmol. 2008; 92: 210-2. Krishnan R et al. Clin Exp Ophthalmol 2009; 37: 384-8).

As subretinal haemorrhages are, so far, inadequately addressed with the use of the current anti-angiogenic therapies, many different treatments have been used to physically remove or displace them, with dubious results (Zhao L, et al. Am J Pathol. 2011; 179: 1265-77).

Vitreous haemorrhage is a common disease, with varied clinical manifestations and causes. The most common causes include proliferative diabetic retinopathy, vitreous detachment with or without retinal breaks, and trauma. Less common causes include vascular occlusive disease, retinal arterial macroaneurism, hemoglobinopathies, neovascular age-related macular degeneration, intraocular tumors, and others. Haemorrhage into the vitreous gel results in rapid clot formation and is followed by slow clearance of approximately 1% per day (Spraul GW Surv Ophthalmol 1997; 42: 3-39). Complications of nonclearing vitreous haemorrhage are hemosiderosis bulbi and glaucoma. The treatment option for nonclearing vitreous haemorrhage is pars plana vitrectomy. Thus, safe and efficient therapy for SMH and vitreous haemorrhages is needed.

Inventor surprisingly found that a single intravitreal injection of dobesilate is capable to clear submacular and vitreous haemorrhages. A first aspect of the invention refers to the use of dobesilate and its derivatives, its pharmaceutically acceptable salts and solvates in the elaboration of a medicine for the treatment of submacular and vitreous haemorrhages. These and other aspects of the present invention are explained in detail herein.

### ABSTRACT OF THE DISCLOSURE

The invention describes compositions and methods of use for dobesilate and pharmaceutically acceptable salts thereof for treating submacular and vitreous haemorrhages. In the invention the therapeutic agent is diethylamine 2,5-dihydroxybenzene sulphonate.

### DETAILED DESCRIPTION OF THE INVENTION

As used throughout the disclosure, the following terms, unless otherwise indicated, shall be understood to have the following meanings.

"Patient" refers to animals, preferably mammals, most preferably humans, and includes males and females.

"Effective amount" refers to the amount of the compound and/or composition that is effective to achieve its intended purpose.

"Treating"; "treat" or "treatment" refers to using the compound or compositions of the present invention either prophylactically to prevent the symptoms of the disease or disorder, or therapeutically to ameliorate an existing condition.

"Therapeutic agent" includes any therapeutic agent can be used to treat or prevent the disease described herein. Therapeutic agents include, but are not limited to, antioxidants, anti-inflammatory compounds and the like. Therapeutic agent includes the pharmaceutically acceptable salts thereof, pro-drugs and pharmaceutical derivatives thereof.

"Transepithelial" refers to the delivery of a compound by application to the ocular surface.

"Intravitreal" refers to the delivery of a compound by application directly in the vitreous.

"Penetration enhancement" or "permeation enhancement" refers to an increase in the permeability of the epithelial tissue to a selected pharmacologically active compound such that the rate at which the compound permeates through the epithelial tissue is increased.

"Carriers" or "vehicles" refers to carrier materials suitable for compound administration and include any such material known in the art such as, for example, any liquid, gel, cream, solvent, liquid diluents, solubilizer or the like, which is non-toxic and which does not interact with any components of the composition in a deleterious manner.

"Sustained release" refers to the release of an active compound and/or composition that are maintained within a desirable therapeutic range over a period of time. The sustained release formulation can be prepared using any conventional method known to one-skilled in the art to obtain the desired release characteristics.

The compound used in this invention can be synthesized by one skilled in the art using conventional methods or is commercially available. The synthesis of these compounds is disclosed in for example "The Merck Index" 13th edition, Merck & Co, R. Rahway N.J. USA. 2001.

The compound and compositions of the invention can be administered by any available and effective delivery system, including, but not limited to by topical application, subconjunctival application or by intravitreal administration in dosage unit formulations containing conventional non-toxic pharmaceutical acceptable carriers, adjuvant and vehicles as desired.

Topical administration of the compound and compositions invention can be in the form of creams, gels, ointments, dispersions, solid sticks, emulsions, microemulsions and eye drops that can be formulated according to the conventional methods using suitable excipients.

Injectable preparations, for example, sterile injectable aqueous suspensions can be formulated according to the known act using suitable dispersing agents, wetting agents and/or suspending agents. The sterile injectable preparations can also be a sterile injectable solution or suspension in a non-toxic acceptable diluent or solvent. Among the acceptable vehicles and solvents that can be used are water, Ringer's solution, and isotonic sodium chloride solution.

Generally, the dosage required to provide an effective amount of the compound and composition, which can be adjusted by one of ordinary skill in the art, will vary depending on the extent of the disease, frequency of treatment, the route of administration, pharmacological considerations such as the activity, efficacy, pharmacokinetic and toxicology profiles of the compound, whether a drug delivery system is used, and whether the compound is administered as part of a drug combination.

The amount of dobesilate that will be effective in the treatment of submacular and vitreous haemorrhages can be determined by standard clinical techniques, including reference to Goodman and Gilman supra: the Physician's Desk Reference, Medical Economics Company, Inc.; Oradell N.J. 1995; and Drug Facts and Comparisons; Inc. St. Louis, MO, 1993. The precise dose to be used in the formulation will also depend on the route of administration, and the seriousness of the disease or disorder, and should be decided by the physician and the patient's circumstances.

### EXAMPLES

The following non-limiting example further describes and enables one of ordinary skill in the art to make and use the present invention.

### Example 1. Effect of dobesilate in submacular haemorrhage

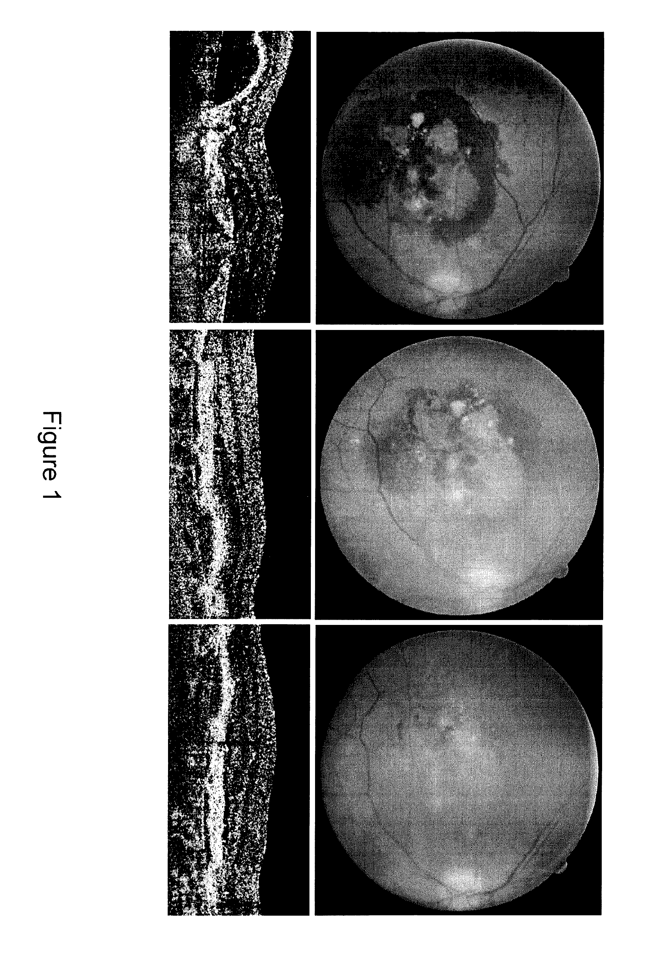

A 75 year-old man with a history of wet age-related macular degeneration (AMD) in both eyes developed a sudden vision decrease in the right eye. The best corrected visual acuity (BCVA) on presentation was of 0.05. Fundus examination showed a recent medium-sized submacular haemorrhage (SMH) (Fig. 1A), and spectral-domain optical coherence tomography (SD-OCT) depicted a remarkable anomalous architecture of the retina and serious disturbances under the retinal pigment epithelium (RPE) without significant accumulation of intraretinal fluid (Fig. 1D). Haemorrhage seems also result in the RPE detachment at some points (Fig 1D, asterisk). Intravitreal dobesilate was administered in the right eye, using a standard protocol. After approval of our Institution Ethical Committee, the patient signed an informed consent form, which included a comprehensive description of dobesilate and the proposed procedure. Patient received an intravitreal solution of dobesilate (150µl) under sterile conditions in his right eye, following the International Guidelines for intravitreal injections (Aiello LP et al. Retina. 2004; 24 (5 Suppl):S3-S19). Dobesilate was administered as a 12.5% solution of diethylammonium 2.5-dihydroxybenzenesulfonate (etamsylate; dicynone® Sanofi-Aventis. Paris. France). No ocular side effects were observed upon the administration of dobesilate or during the following days.
Along two months follow-up the haemorrhage signs gradually disappeared (Fig 1B, C), the SD-OCT scans showed a progressive normalization of the retinal architecture with disappearance of RPE detachment (Fig. 1E, F) and the final BCVA became 0.50.

### Example 2- Effect of dobesilate in vitreous haemorrhage

A 68 old-year type 2 diabetic man presented with intense vision loss in his right eye. Ophthalmic biomicroscopic examination showed a pseudophakic right eye with non-recent and recent haemorrhages in the anterior segment (Figure 1A). Fundoscopic study revealed a massive vitreous haemorrhage (Figure 1B). Intraocular pressure was 50mmHg that was reduced to 14mmHg after a paracentesis procedure. After approval of the Institutional Ethics Committee, the patient signed an informed consent form, which included a comprehensive description of dobesilate and the proposed procedure. Patient received an intravitreal solution of dobesilate (150 µl) under sterile conditions, following the International Guidelines for intravitreal injections (Aiello LP et al. Retina. 2004; 24 (5 Suppl):S3-S19) in his right eye. Dobesilate was administered as a 12.5% solution of diethylammonium 2.5-dihydroxybenzenesulfonate (etamsylate, dicynone® Sanofi-Aventis. Paris. France). No ocular side effects were observed upon the administration of dobesilate or during the following days. As soon as three days after treatment haemorrhage was completely cleared in the anterior segment (Fig 1B) although still incompletely in the vitreous cavity (Fig 1C). Intraocular pressure was maintained at normal level.

### BRIEF DESCRIPTION OF FIGURES

**Figure 1** Sequence of fundus photographs (A-D) and spectral-domain optical coherence tomography (SD-OCT) scans (D-F) of a patient with a moderate-sized submacular haemorrhage at baseline (A,D), and one (B,E) and two months (C,F) after a single intravitreal injection of dobesilate. Asterisk in D indicates retinal pigment epithelium (RPE) detachment.

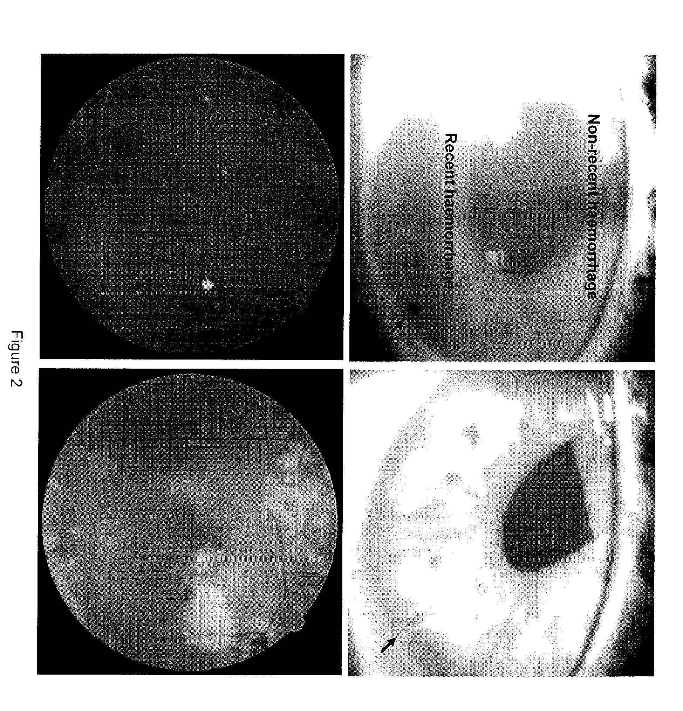

**Figure 2** Efficacy of intravitreal dobesilate injection to clear vitreous haemorrhage in a diabetic patient. A and C photographs were taken at presentation, and B and D after 3 days of treatment. Non-.recent and recent haemorrhages viewed at presentation (A) resolved after treatment (B). At presentation (C) fundoscopy showed a view to the retina being blocked by blood. After treatment (D) a clear view to the retina with residual blood temporally was depicted. Note in B the abnormal appearance of the pupil due to luxation of the lens. Arrow indicates paracentesis site.

## Claims

1. A method for treating patients with submacular and vitreous haemorrhages using a therapeutically effective amount of dobesilate or pharmaceutically acceptable salts and esters.

2. The method of claim 1 wherein administered compound is diethylamine 2,5-dihydroxybenzenesulfonate.

3. The method of claim 1 wherein the therapeutic agent can be administered into the ocular surface or for invasive method as such intravitreal application

4. The method of claim 1 wherein the therapeutic agent can be administered as any method using a sustained release of active compound.

5. The method of claim 1 wherein the therapeutic agent can be used for treating haemorrhages of the eyes as occurred in proliferative diabetic retinopathy, vitreous detachment with or without retinal breaks, trauma, vascular occlusive disease, retinal arterial macroaneurism, hemoglobinopathies, neovascular age-related macular degeneration, intraocular tumors and others.
